# EUROPEAN PATENT SPECIFICATION

(11) **EP 3 695 775 B1**
(45) Date of publication and mention of the grant of the patent: **19.07.2023**
(21) Application number: 20154207.3
(22) Date of filing: 28.01.2020
(51) Int. Cl.: A61B 3/00, A61B 3/12, A61B 3/15

(54) **SMARTPHONE-BASED HANDHELD OPTICAL DEVICE AND METHOD FOR CAPTURING NON-MYDRIATIC RETINAL IMAGES**
SMARTPHONE-BASIERTE TRAGBARE OPTISCHE VORRICHTUNG UND VERFAHREN ZUR ERFASSUNG VON NICHT-MYDRIATISCHEN RETINALEN BILDERN
DISPOSITIF OPTIQUE PORTATIF BASÉ SUR UN TÉLÉPHONE INTELLIGENT ET PROCÉDÉ DE CAPTURE D'IMAGES RÉTINIENNES NON MYDRIATIQUES

(30) Priority: 18.02.2019 PT 2019115320; 27.06.2019 EP 19183057
(43) Date of publication of application: 19.08.2020
(73) Proprietor: Associação Fraunhofer Portugal Research, 4200-135 Porto (PT)
(72) Inventor: GOMES SOARES, Filipe Cruz, 4250-095 Porto (PT); CASTRO GONÇALVES, João Nuno, 5470-333 Outeiro MTR (PT); DOS SANTOS DA ROCHA FELGUEIRAS, Simão, 4900-496 Viana do Castelo (PT); PEREIRA FERREIRA PEIXOTO, Ricardo Felipe, 4410-133 São Felix da Marinha (PT); SOARES TOJAL DE MENESES, Ricardo Manuel, 4485-101 Fajozes (PT); SIMÕES DE MELO, David, 2620-520 Ramada (PT)
(74) Representative: Patentree

(56) References cited:
- WO-A1-2017/180965
- US-A1- 2017 280 996
- US-A1- 2019 028 634
- US-B1- 9 462 945

## Description

### TECHNICAL FIELD

The present disclosure relates to the field of decentralised screening in ophthalmology, with an apparatus for screening the human retina towards the diagnosis of, but not limited to: Diabetic Retinopathy (DR), Glaucoma, Age-Related Macular Degeneration, Cardiovascular diseases, Cerebrovascular diseases.

### BACKGROUND

There are 217 million people in the world with moderate to severe vision impairment, while Age-related Macular Degeneration, Glaucoma and Diabetic Retinopathy (DR) are among the top causes. DR may lead to complete vision loss, being related to the systemic nature of diabetes, as the glycaemic control and hypertension. To prevent its progression, extensive screening actions should be performed.

The increasing prevalence of diabetes in the population is associated with several health issues, one of which is the development of DR. This disease causes several cumulative lesions to the patient retina through microvascular changes and, when untreated, may lead to blindness. DR progression is mostly asymptomatic until the later stages of the disease, when vision loss settles in, and at that point it is very difficult to reverse. Around 50% of all the diabetic patients for more than ten years exhibit some stage of DR. The recommendation for yearly retinal imaging of the diabetic population is often not followed, due to high costs and lack of trained personnel.

Glaucoma is the second leading cause of blindness globally and its prevalence is projected to increase with population growth and the aging of the population. Since nowadays the diagnosis of Glaucoma mainly occurs after the opportunistic evaluation of ocular tension, Glaucoma is still underdiagnosed. Thus, the structural analysis by retinal imaging is crucial to cover most forms of Glaucoma, including Normal-Pressure Glaucoma. For this reason, a population-based screening programme for Glaucoma is currently being advocated by groups of specialists in Portugal, but screening programmes are generally missing all over the world.

Currently, the aforementioned problem is solved neither by the conventional tabletop fundus cameras nor by the more portable solutions recently entering the market. The existing forms of handheld non-mydriatic fundus cameras have several issues hindering a decentralised and widely available screening in ophthalmology: they are still expensive; only offer simple or none image processing and quality validation during image acquisition; require highly trained personnel for operation; do not give access to transfer or copy the acquired images to external non-proprietary systems; and, most of all, they require ophthalmology experts to manually analyse the obtained images, data storage and reading centre services. Some solutions offer cloud services for proprietary DR assessment which, unlike advertised, are not suitable for all professional fundus cameras, or are only focused on early stages of DR leaving off the radar important lesions as exudates. An existing handheld optical device is disclosed in prior art document US2019/028634. The handheld optical device disclosed therein comprises a smartphone having a camera, an inertial sensor and an electronic data processor wherein it uses an image classification method configured by pre-training to analyze the images and provide instructions to the user to move the camera in order to properly align the camera with the retina visual axis. Prior art document WO2017/180965 also relates to a portable retinal imaging apparatus comprising an optical system for coupling to a camera equipped with a smartphone and an inertial sensor. Document US9462945 discloses an apparatus for performing automatic retinal screening wherein iamge quality is determined based on the presence and extent of shadows in the image.

These facts are disclosed in order to illustrate the technical problem addressed by the present disclosure.

### GENERAL DESCRIPTION

The present disclosure relates to the field of decentralised screening in ophthalmology, with an apparatus for screening the human retina towards the diagnosis of, but not limited to: Diabetic Retinopathy (DR), Glaucoma, Age-Related Macular Degeneration, Cardiovascular diseases, Cerebrovascular diseases. In particular, it relates to the technical problem of proposing a solution for mobile, viable, low-cost, accessible and non-mydriatic retinal imaging (without drug-induced pupil dilation) that can be massively available and usable by non-experts in ophthalmology to acquire images with laboratory-grade quality. Moreover, from the data obtained by the optical device, (a fundus camera), a system for computer-aided diagnosis is expected to contribute for the improvement of eye health by the detection of unscreened or poorly screened diseases in the world population.

A fundus camera is a complex optical system used for imaging the retina of the eye. Retinal imaging presents a unique difficulty considering that the retina must be illuminated and imaged simultaneously, a process which forces illumination and imaging systems to share a common optical path. While patent literature shows design forms, these patents lack specification for the optical layout, such as lens power, spacing, and conjugate planes. Consequently, performing illumination and imaging analysis simulation of these designs are practically impossible.

The present disclosure for eye screening comprising a 3D-printed and affordable optical device that can be operated by non-experts in ophthalmology. One of the major goals is to improve patient access to early treatment, to empower non-experts to acquire retinal images and decrease the burden of screening actions on healthcare systems.

The asymptomatic initial progression of DR and the high effectiveness of early treatment have motivated the implementation of extensive screening programs covering the diabetic population, in which multiple images of the patient retinas are acquired by trained personnel and subsequently manually analysed, one by one, by an expert in ophthalmology. The present disclosure addresses these two issues, the asymptomatic initial progression of eye diseases and the complex implementation of screening actions, by presenting a self-contained mobile-based solution. The technology comprises the automated and early detection of eye diseases with a low-cost optical device attached to a smartphone for handheld and guided retinal image acquisition.

Increasingly capable smartphones enable this disclosure, by providing a mobile computational platform with relevant imaging features. The automated analysis is able to provide a first assessment on the presence of DR, by employing advanced machine learning algorithms to process the image in search of lesions such as microaneurysms and exudates, often associated with the onset of DR.

Some of the most important advantages of the present disclosure, comprises:
the control and simplification of the handheld retinal imaging, by intelligent guidance and evaluation of the right moment for auto-capture, thanks to Al that enables the recognition of the scene during preview frames;
the reliability of computer vision algorithms is strongly related with the quality of the images acquired. The consistency of handheld capture deeply depends on proper human interaction, which is frequently neglected. In order to tackle these challenges, real-time computer vision combined with inertial sensors on the smartphone provide crucial data for dedicated GUI for effective Human Computer Interaction during the retinal alignment;
mobile-based and powerful central processing unit allows real-time guidance, camera control, light control and image processing in parallel, besides the computer-aided diagnosis of each clinical case, all offline and on-device;
a physical button on device to trigger the acquisition start, without the need for touching the smartphone during the acquisition process;
mechanical adjustment of dioptre range plus auto-focusing system;
alignment and acquisition mode on the same sensor with three light sources (two lights and one matrix of internal fixation targets) controlled by dedicated printed circuit board and firmware for light control having two distinct power-sources;
affordable and safe, handheld ophthalmic device according to ISO 15004-2:2007 (Ophthalmic instruments -- Fundamental requirements and test methods -- Part 2: Light hazard protection), fully compliant with ISO 10940:2009 (Ophthalmic instruments - Fundus camera);
device enables screenings in remote areas, improving coverage and frequency of screenings, being prepared for decision support or computer-aided diagnosis of multiple diseases thanks to supervised and multimodal classification methods.

By testing a system with the same smartphone and camera model, under laboratory conditions where it is performed calibration against standard test targets and human eye models. By following a list of verification points of features in GUI. By completely dismantling a device to confirm matches with hardware design and specification and by testing performance features.

Algorithms can be applied to problems requiring handheld image acquisition with quality control for automated visual inspection by machines or manual inspection by humans. The disclosed device can be used by general practitioners or endocrinologists, or by other health professionals non-experts in ophthalmology as nurses.

New forms of human computer interaction can be applied based on real-time data from multiple types of sensors or data sensor fusion. Image acquisition device with variable close-up range applications as macro photography with synchronous or alternating dual wavelength light.

Computer-aided detection and diagnosis algorithms can be generalized for other diseases or inspection problems, every time the texture, geometry and color features in images are relevant, in the visible or invisible spectrum.

According to the invention, a method of operation of a handheld optical device for capturing non-mydriatic retinal images from a patient is disclosed using a smartphone having a camera, an inertial sensor and an electronic data processor, said device comprising a housing for coupling to the smartphone and an optical system for capturing retina images through said camera, said method comprising, for aligning the camera with the retina visual axis, the electronic data processor carrying out the steps of:
capturing an image of the retina;
using an image classification method configured by pre-training on previously captured images to detect whether the camera is lower or higher than the retina visual axis, by detecting whether a dark shadow is present on the top or on the bottom, respectively, of the captured image;
if the camera was detected as being lower or higher than the retina visual axis, then inputting the value of the Y-axis component of the inertial sensor;
if the camera was detected as being lower than the visual axis and the Y-axis value is levelled, instructing the user to translationally move up the device;
if the camera was detected as being lower than the visual axis and the Y-axis value is not levelled, instructing the user to tilt up the device;
if the camera was detected as being higher than the visual axis and the Y-axis value is levelled, instructing the user to translationally move down the device; and if the camera was detected as being higher than the visual axis and the Y-axis value is not levelled, instructing the user to tilt down the device.

In an embodiment, said device comprises a NIR, near-infrared, light source and a visible white light source, and said method further comprises using said NIR light source for non-miotic fundus illumination while aligning the camera with the retina visual axis and comprises using said visible white light source for fundus illumination for retina image acquisition when the camera is aligned with the retina visual axis.

An embodiment further comprises using an image classification method configured by pre-training on previously captured images to detect whether the camera is aligned with the retina visual axis; and triggering the retina image capture when the alignment is detected.

An embodiment further comprises using an image classification method configured by pre-training on previously captured images to detect whether the camera is misaligned to the left or to the right of the retina visual axis, by detecting whether a dark shadow is present on the left or on the right, respectively, of the captured image;
if the camera was detected as being misaligned to the left, instructing the user to rotate the device to the right; and
if the camera was detected as being misaligned to the right, instructing the user to rotate the device to the left.

An embodiment further comprises using an image classification method configured by pre-training on previously captured images to detect whether the camera is too far or too close from the retina, by detecting whether a too small fundus image or a top white spot, respectively, is present in the captured image;
if the camera was detected as being too close to the retina, instructing the user to move the device away from the retina; and
if the camera was detected as being too far from the retina, instructing the user to move the device closer to the retina.

In an embodiment, the pre-training on previously captured images of the image classification method is a machine-learning method.

According to the invention, also a handheld optical device for capturing non-mydriatic retinal images from a patient is disclosed using a smartphone having a camera, an inertial sensor and an electronic data processor, said device comprising said smartphone, a housing for coupling to the smartphone and an optical system for capturing retina images through said camera, said smartphone comprising a non-volatile data carrier comprising instructions readable by said electronic data processor that when executed cause the processor to carry out the following steps for aligning the camera with the retina visual axis:
capturing an image of the retina;
using an image classification method configured by pre-training on previously captured images to detect whether the camera is lower or higher than the retina visual axis, by detecting whether a dark shadow is present on the top or on the bottom, respectively, of the captured image;
if the camera was detected as being lower or higher than the retina visual axis, then inputting the value of the Y-axis component of the inertial sensor;
if the camera was detected as being lower than the visual axis and the Y-axis value is levelled, instructing the user to translationally move up the device;
if the camera was detected as being lower than the visual axis and the Y-axis value is not levelled, instructing the user to tilt up the device;
if the camera was detected as being higher than the visual axis and the Y-axis value is levelled, instructing the user to translationally move down the device;
if the camera was detected as being higher than the visual axis and the Y-axis value is not levelled, instructing the user to tilt down the device.

An embodiment comprises a NIR, near-infrared, light source for non-miotic fundus illumination while aligning the camera with the retina visual axis.

An embodiment comprises a visible white light source for fundus illumination for retina image acquisition.

An embodiment comprises a visible light source matrix arranged for providing visual fixation points for the patient through said optical system.

An embodiment comprises a lens having one planar surface and one convex surface for condensing light rays coming from the visible light source matrix into said optical system.

An embodiment comprises a fixation beam-splitter for separating said visual fixation points from the NIR and visible illumination light sources.

An embodiment comprises a beam-splitter for separating said the NIR and visible illumination light sources from the image capture camera.

An embodiment comprises an aspheric lens for image focusing while avoiding optical aberrations.

An embodiment comprises an ocular lens having both lens surfaces curved.

An embodiment comprises an iris for limiting the captured light by said optical system to the light coming from the eye fundus.

An embodiment comprises one or more light polarizers.

In an embodiment, said housing is obtained by additive manufacturing, in particular 3D printed.

In an embodiment, said housing is 3D printed to accommodate a specific (an individual) smartphone shape.

### BRIEF DESCRIPTION OF THE DRAWINGS

The following figures provide preferred embodiments for illustrating the disclosure and should not be seen as limiting the scope of invention.
**Figure 1****:** Schematic representation of an embodiment of the hardware architecture of the disclosed device.
**Figure 2****:** Schematic representation of an embodiment of the Software architecture of the disclosed device running in the smartphone processor.
**Figure 3****:** Schematic representation of an embodiment of the Optical system of the disclosed device.
**Figure 4****:** Schematic representation of an embodiment of the Optical system of the disclosed device.
**Figure 5****:** Schematic representation of an embodiment of the Cut view of mechanical design of the disclosed device.
**Figure 6****:** Schematic representation of an embodiment of the Cut view of mechanical design of the disclosed device.
**Figure 7****:** Schematic representation of an embodiment of the Perspective view of mechanical design of the disclosed device.
**Figure 8****:** Schematic representation of an embodiment for handheld movements the user can perform while handling the disclosed device.
**Figure 9****:** Schematic representation of an embodiment of the X-axis rotation (Panning).
**Figure 10****:** Schematic representation of an embodiment of the Z-axis rotation (Roll).
**Figure 11****:** Schematic representation of an embodiment of the Y-axis rotation (Tilting rotation).
**Figure 12****:** Schematic representation of an embodiment of the Computer vision algorithm by fusion of mage and inertial sensors.

### DETAILED DESCRIPTION

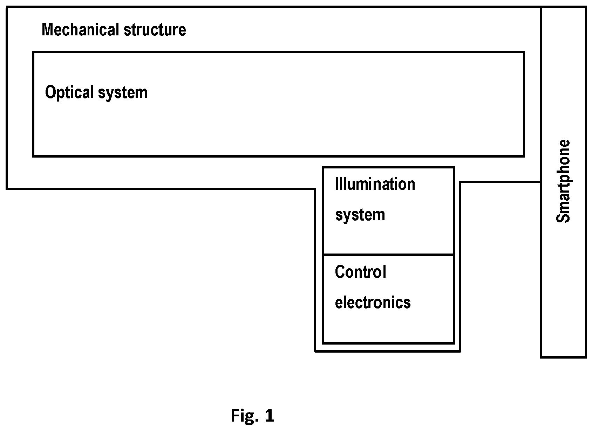

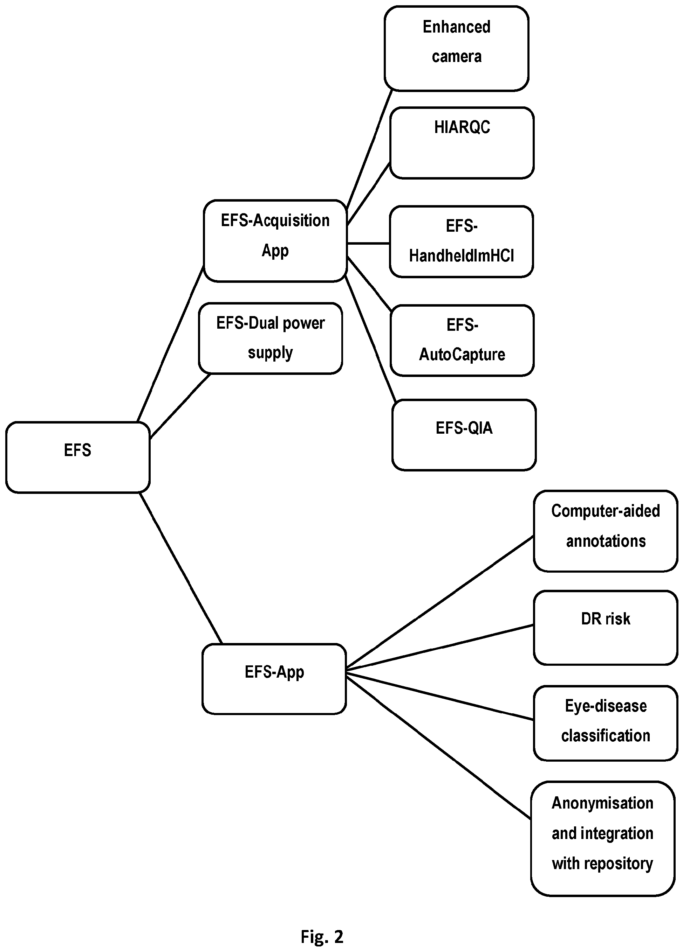

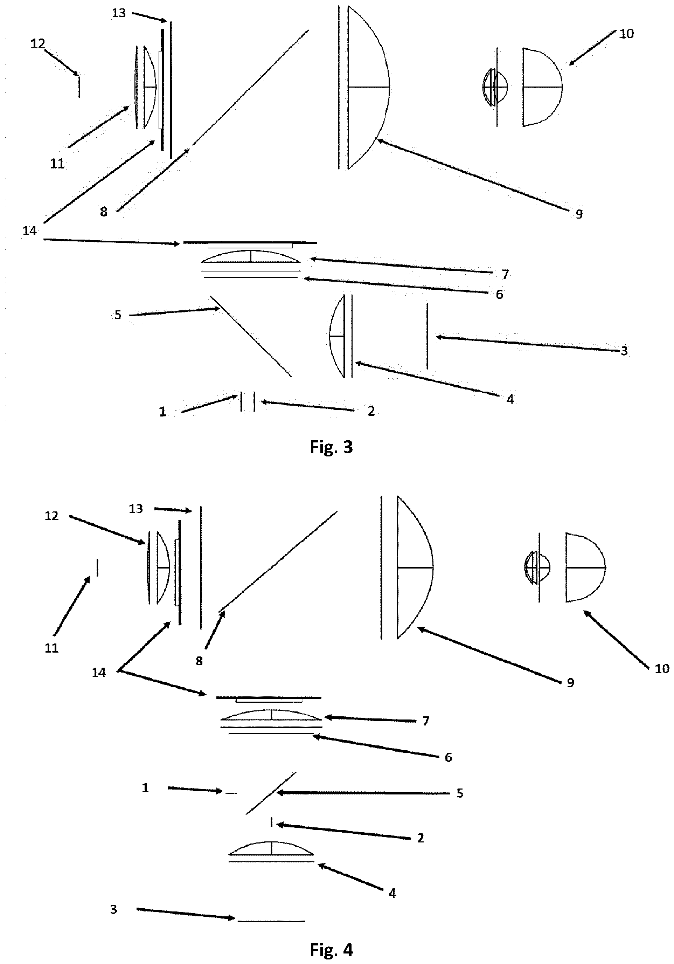

Figure 1 illustrates a schematic representation of an embodiment of the hardware architecture of the disclosed device. Figure 2 illustrates a schematic representation of an embodiment of the Software architecture of the disclosed device running in the smartphone processor. Figure 3 illustrates a schematic representation of an embodiment of the Optical system of the disclosed device, where:
(1) represents the NIR LED - Light Source for Fundus Illumination. To be on during the guide mode, to allow the examiner to search for the better image, without provoking patient mydriasis;
(2) represents the white LED - Light Source for Fundus illumination. To be on during the acquisition of the image;
(3) represents the Miniature LED Matrix - Light Source for Internal Fixation Points. This item ensures patient eye fixation during the exam at the same time that allows observation of several regions of the posterior pole of the eye. By turning on a specific set of LEDs at a time, will force the patient to move is eye and consequently allow the observation of more peripheral parts of the retina;
(4) represents the plano-Convex Condenser Lens - Lens with one planar and one convex surface, used to condensate light rays coming from the LED matrix in order to reach the optical component 5;
(5) represents the Beamsplitter - This component allows the transmission of a percentage of light at the same time that reflects the rest. It is available with several different Reflection/Transmission ratios. In this embodiment it is used to separate the internal fixation point light path from illumination paths (both NIR and White);
(6) represents the iris - The iris or diaphragm is a piece with a hole in the middle that only allows the passage of the needed light to illuminate the fundus. Helpful for reflection prevention;
(7) represents the plano-Convex Condenser Lens - Lens used to condensate light rays originating from the light source. May be equal to element 4;
(8) represents the plate Beamsplitter - This component allows the transmission of a percentage of light at the same time that reflects the rest. In this specific embodiment it is used to separate the illumination path from the imaging path;
(9) represents the aspheric Objective Lens - Lens that focus the rays on the pupil in order to allow fundus illumination. At least one of the surfaces presents an aspherical shape in order to prevent spherical aberrations and allow the maximum possible light rays to pass the pupil;
(10) represents the human eye;
(11) represents the Best Form Ocular Lens - Lens having a radius of curvature on both surfaces that when conjugated minimizes prevent spherical aberrations. It is used as an ocular lens and has the main goal of leading the image formed by element 9 to the smartphone camera. Depending on the camera focal length, a close-up or macro filter can be placed next to 11 to extend the close-up range of a digital camera;
(12) represents the digital Image Sensor - Camera with suitable resolution and with good setting control, allowing a good quality image of the retina;
(13) represents the Iris - The iris or diaphragm is a piece with a hole in the middle that only allows the passage of the needed light to illuminate the fundus. Helpful for reflection prevention only allowing rays coming from the fundus to reach the smartphone camera;
(14) represents the polarizers - Polarizers are, by definition, optical filters where the transmission of light is dependent on its polarization. Being helpful to block light with a specific polarization having many applications in fields like microscopy and photography. Taking advantage of the polarization state changes light suffers along the optical path, polarizers can be important to prevent undesired reflections from reducing the quality of the final image obtained, ensuring that light mostly coming from the fundus reaches the digital image sensor.

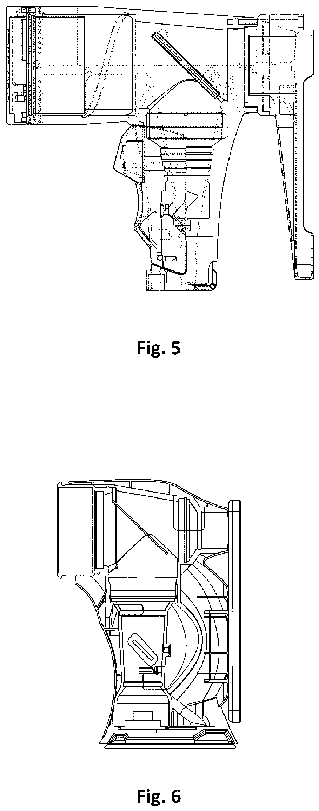

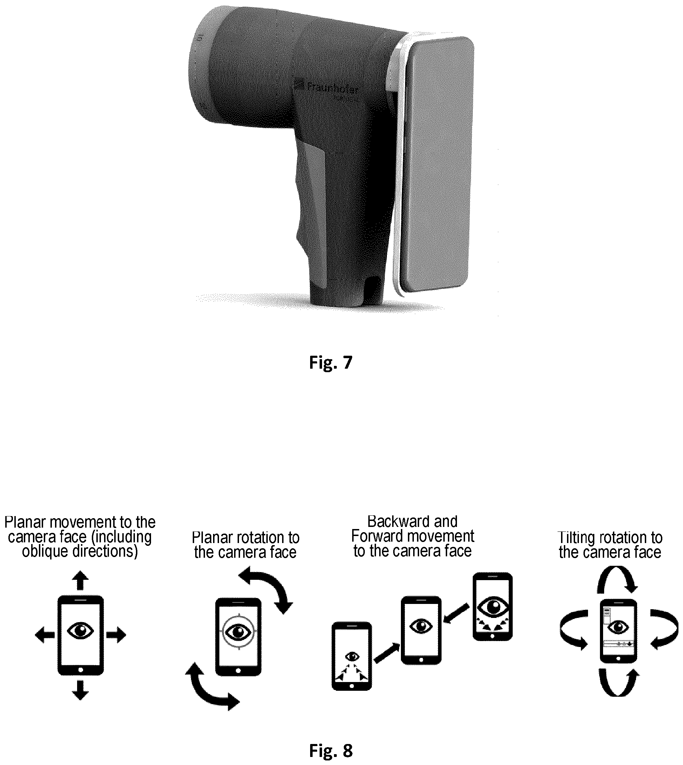

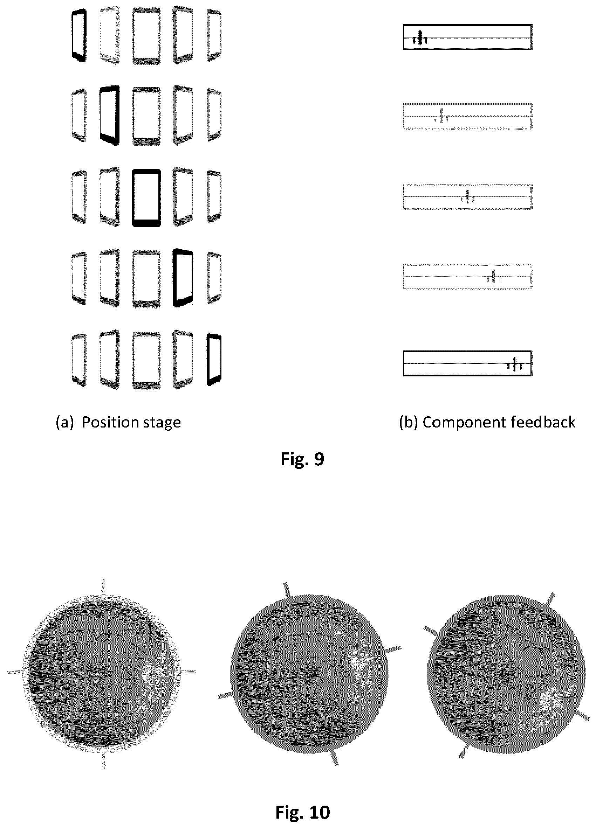

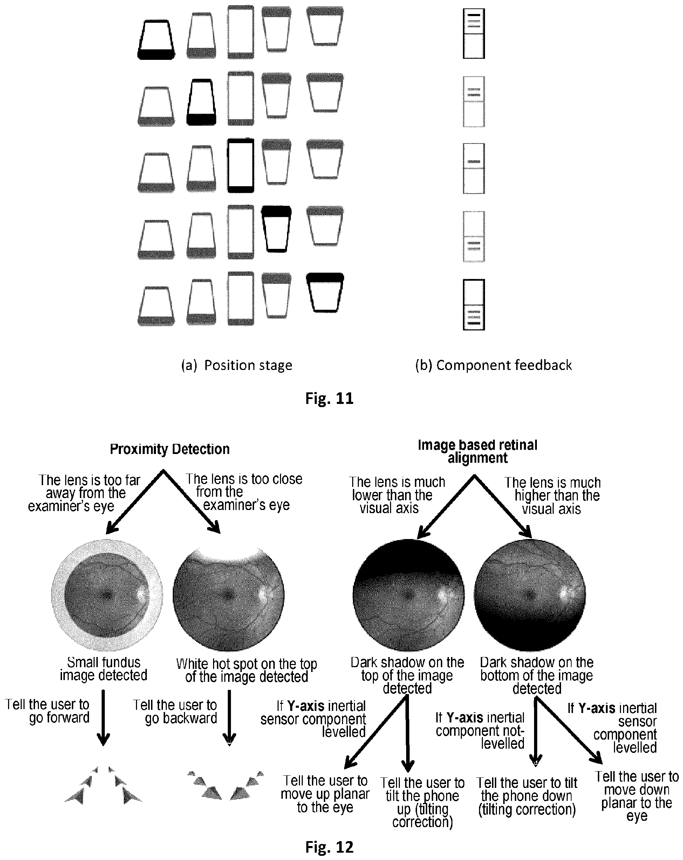

Figure 4 illustrates another embodiment of the Optical System in which the Miniature LED Matrix **3** and the plano-Convex Condenser Lens **4** are placed below the White LED **2.** The Beamsplitter **5** in this embodiment is used to separate the infrared light coming from the NIR LED **1** and the white light coming from the white LED **2.** Figure 5 illustrates a schematic representation of an embodiment of the Cut view of mechanical design of the disclosed device. Figure 6 illustrates a schematic representation of another embodiment of the Cut view of mechanical design of the disclosed device. Figure 7 illustrates a schematic representation of an embodiment of the Perspective view of mechanical design of the disclosed device. Figure 8 illustrates a schematic representation of an embodiment of some of the handheld movements the user can perform while handling the disclosed device. Figure 9 illustrates a schematic representation of an embodiment of the X-axis rotation (Panning). Figure 10 illustrates a schematic representation of an embodiment of the Z-axis rotation (Roll). Figure 11 illustrates a schematic representation of an embodiment of the Y-axis rotation (Tilting rotation). Figure 12 illustrates a schematic representation of an embodiment of the Computer vision algorithm by fusion of mage and inertial sensors.

The present disclosure comprises a mobile application that employs parallel computation and artificial intelligence (Al) offline, to control and simplify the handheld image acquisition of the retina through real-time user guidance and evaluation of the right moment for auto-capture. Computer-Aided Diagnosis running on-device can be used to rate the risk level of DR, visualize computer-detected annotations of exudates and microaneurysms, and classify them between pathology-free and eye-disease cases.

The present disclosure technology aims to simplify, anticipate and increase the coverage of retinal screening as a powerful image acquisition and pre-diagnostic tool. Currently, the disclosure technology comprises: a low-cost, 3D-printed and handheld non-mydriatic fundus camera device with the disclosed device optical and mechanical design coupled with:
a. dedicated electronics and firmware for light control and dual power-supply (EFS-Dual power supply), allowing continuous acquisition and processing in rural scenarios/ICT4D, even on smartphones that do not support charging and power delivery at the same time. A Smartphone Calibration App was developed to expand the compatibility list of smartphone models.
b. a smartphone combined with the disclosed mobile image processing software taking advantage of the faster and more efficient processing units increasingly available, by empowering:
   i. AI software for user guidance based on sensor fusion and dedicated Graphical User Interface (GUI) for effective Human Computer Interaction during the retinal alignment;
   ii. the evaluation of the right moment for auto-capture (EFS-AutoCapture). This evaluation is performed by analysing, in real time, the stream of frame previews by comparing their colour with retinal templates while vessels or optic disc are present. The implementation automatically triggers an acquisition whenever a fixed number of consecutive preview frames are considered as retina.
   iii. quality control (EFS-QIA) with quick feedback when the user should repeat the acquisition;
   iv. The central processing unit of the apparatus is the smartphone, but this invention disclosure includes two options for digital image sensors: smartphone built-in camera or embedded camera precisely placed inside the mechanical prototype.

The disclosure also includes EFS-App: a mobile application that employs parallel computation (all of this can be done offline):
a. to rate the DR risk level based on number, type and size of structures;
b. to visualize computer-aided annotations of exudates and microaneurysms automatically detected;
c. to classify between pathology-free and DR cases, based on Al software for telemedicine and decision-support use cases.

The disclosure also includes the use of an anonymized data repository of mobile acquisitions, and a web portal for quick image annotation and classification by specialists (ground truth generator).

Alternatively, the disclosure software is compatible with, for example, Welch Allyn PanOpticTM ophthalmoscope via adapter and dedicated light source that can be operatively coupled with the smartphone processor. However, the field-of-view is much smaller and some features such as non-mydriatic acquisition or improved usability are not possible. In this type of images, image stitching [8] can be applied to combine multiple images for a wider composed view. In addition, the disclosure technology has the potential to be extended to other eye diseases and stand as a useful tool for eye health screening in developing countries where even lower number of specialists in Orthoptics and Ophthalmology are available.

The disclosed device (Figure 4 and 6) can be a handle-equipped device designed to be extremely portable, lightweight and comfortable to hold. The mechanical structure is composed by removable electronics and illumination system, being easy to maintain and adapt. Dynamic focusing system allows control of a plurality of different dioptre ranges.

The software for effective Human Computer Interaction during the retinal alignment while using the handheld device (EFS-HandheldlmAHCl) is described below.

The Inertial Sensor based graphical components were created in order to give the user a proper indication of the handling of the smartphone. The 3-axis were taken into consideration for the inertial sensor based components.

The X-axis component appears as a horizontal bar giving the user a proper indication of the alignment when the user stands or sits in front of the patient. This will help the user to keep the lenses in line with the examined eye.

The Z-axis component (outer circle) assures that the smartphone is properly aligned with the typical vertical position of the patient. By standardizing the orientation of all acquired images, it allows an easier detection of the quadrant on which the lesions appear. Furthermore, the panoramic image achieved when using the internal fixation points and the stitching method will also be facilitated.

Y-axis components will indicate when the smartphone is lower or higher than the visual axis. The purpose is to guide the user to keep the lenses levelled with the examined eye.

The disclosed EFS App is previously trained to distinguish between retinal images with shadows, following supervised machine learning. Once the EFS App is able to recognize hotspots, low FOV, or dark shadows that appear on the borders of the image (Image based retinal alignment) and after combining it with the feedback given by the inertial sensor components (aforementioned), the user should have a clear idea of how to correct the alignment either by moving the smartphone planar to the eye or by adjusting it with a tilting movement.

Figure 10 shows different states for recognition by computer vision and Al. For the image based retinal alignment, the observed scenes are just an example. The correct alignment is achieved when the camera is in perpendicular, parallel and coaxial to the patient eye.

The term "comprising" whenever used in this document is intended to indicate the presence of stated features, integers, steps, components, but not to preclude the presence or addition of one or more other features, integers, steps, components or groups thereof. The disclosure should not be seen in any way restricted to the embodiments described and a person with ordinary skill in the art will foresee many possibilities to modifications thereof. The above described embodiments are combinable. The following claims further set out particular embodiments of the disclosure.

## Claims

1. Method of operation of a handheld optical device for capturing non-mydriatic retinal images from a patient using a smartphone having a camera, an inertial sensor and an electronic data processor, said device comprising a housing for coupling to the smartphone and an optical system for capturing retina images through said camera, said method comprising, for aligning the camera with the retina visual axis, the electronic data processor carrying out the steps of:
capturing an image of the retina;
using an image classification method configured by pre-training on previously captured images to detect whether the camera is lower or higher than the retina visual axis, by detecting whether a dark shadow is present on the top or on the bottom, respectively, of the captured image;
if the camera was detected as being lower or higher than the retina visual axis, then inputting the value of the Y-axis component of the inertial sensor;
if the camera was detected as being lower than the visual axis and the Y-axis value is levelled, instructing the user to translationally move up the device;
if the camera was detected as being lower than the visual axis and the Y-axis value is not levelled, instructing the user to tilt up the device;
if the camera was detected as being higher than the visual axis and the Y-axis value is levelled, instructing the user to translationally move down the device; and if the camera was detected as being higher than the visual axis and the Y-axis value is not levelled, instructing the user to tilt down the device.

2. Method according to the previous claim, wherein said device comprises a NIR, near-infrared, light source and a visible white light source, and said method further comprises using said NIR light source for non-miotic fundus illumination while aligning the camera with the retina visual axis and comprises using said visible white light source for fundus illumination for retina image acquisition when the camera is aligned with the retina visual axis.

3. Method according to according to any of the previous claims further comprising:
using an image classification method configured by pre-training on previously captured images to detect whether the camera is aligned with the retina visual axis; and
triggering the retina image capture when the alignment is detected.

4. Method according to according to any of the previous claims further comprising using an image classification method configured by pre-training on previously captured images to detect whether the camera is misaligned to the left or to the right of the retina visual axis, by detecting whether a dark shadow is present on the left or on the right, respectively, of the captured image;
if the camera was detected as being misaligned to the left, instructing the user to rotate the device to the right; and
if the camera was detected as being misaligned to the right, instructing the user to rotate the device to the left.

5. Method according to according to any of the previous claims wherein the pre-training on previously captured images of the image classification method is a machine-learning method.

6. Handheld optical device for capturing non-mydriatic retinal images from a patient using a smartphone having a camera, an inertial sensor and an electronic data processor, said device comprising said smartphone, a housing for coupling to the smartphone and an optical system for capturing retina images through said camera, said smartphone comprising a non-volatile data carrier comprising instructions readable by said electronic data processor that when executed cause the processor to carry out the following steps for aligning the camera with the retina visual axis:
capturing an image of the retina;
using an image classification method configured by pre-training on previously captured images to detect whether the camera is lower or higher than the retina visual axis, by detecting whether a dark shadow is present on the top or on the bottom, respectively, of the captured image;
if the camera was detected as being lower or higher than the retina visual axis, then inputting the value of the Y-axis component of the inertial sensor;
if the camera was detected as being lower than the visual axis and the Y-axis value is levelled, instructing the user to translationally move up the device;
if the camera was detected as being lower than the visual axis and the Y-axis value is not levelled, instructing the user to tilt up the device;
if the camera was detected as being higher than the visual axis and the Y-axis value is levelled, instructing the user to translationally move down the device;
if the camera was detected as being higher than the visual axis and the Y-axis value is not levelled, instructing the user to tilt down the device.

7. Device according to the previous claim comprising a NIR, near-infrared, light source for non-miotic fundus illumination while aligning the camera with the retina visual axis.

8. Device according to according to any of the claims 6 - 7 comprising a visible white light source for fundus illumination for retina image acquisition.

9. Device according to according to any of the claims 6 - 8 comprising a visible light source matrix arranged for providing visual fixation points for the patient through said optical system.

10. Device according to the previous claim comprising a lens having one planar surface and one convex surface for condensing light rays coming from the visible light source matrix into said optical system.

11. Device according to according to claims 7, 8 and any of the claims 9 - 10 comprising a beam-splitter for separating said visual fixation points from the NIR and visible illumination light sources.

12. Device according to according to any of the claims 6 - 11 comprising a beam-splitter for separating said the NIR and visible illumination light sources from the image capture camera.

13. Device according to according to any of the claims 6 - 12 comprising:
an aspheric lens for image focusing while avoiding optical aberrations, and/or
an ocular lens having both lens surfaces curved, and/or
an iris for limiting the captured light by said optical system to the light coming from the eye fundus.

14. Device according to according to any of the claims 6 - 13 comprising one or more light polarizers.

15. Device according to according to any of the claims 6 - 14 wherein said housing is 3D printed to accommodate a specific smartphone shape.

## Patentansprüche

1. Verfahren zum Betrieb einer tragbaren optischen Vorrichtung zum Erfassen nicht-mydriatischer Netzhautbilder eines Patienten unter Verwendung eines Smartphones mit einer Kamera, einem Trägheitssensor und einem elektronischen Datenprozessor, wobei die genannte Vorrichtung ein Gehäuse zum Verbinden mit dem Smartphone und ein optisches System zum Erfassen von Netzhautbildern durch die genannte Kamera umfasst, wobei das genannte Verfahren zum Ausrichten der Kamera auf die Sehachse der Netzhaut beinhaltet, dass der elektronische Datenprozessor die folgenden Schritte ausführt:
Erfassen eines Bildes der Netzhaut;
Verwendung eines Verfahrens zur Klassifizierung von Bildern, das durch ein Vortraining mit zuvor erfassten Bildern konfiguriert wurde, um zu erkennen, ob die Kamera über oder unter der Sehachse der Netzhaut liegt, indem erkannt wird, ob auf dem erfassten Bild oben bzw. unten ein dunkler Schatten vorhanden ist;
wurde erkannt, dass die Kamera über oder unter der Sehachse der Netzhaut liegt, wird der Wert der Y-Achsenkomponente des Trägheitssensors eingegeben;
wurde erkannt, dass die Kamera unter der Sehachse liegt und der Wert der Y-Achse nivelliert ist, wird der Benutzer angewiesen, die Vorrichtung translatorisch nach oben zu bewegen;
wurde erkannt, dass die Kamera unter der Sehachse liegt und der Wert der Y-Achse nicht nivelliert ist, wird der Benutzer angewiesen, die Vorrichtung nach oben zu kippen;
wurde erkannt, dass die Kamera über der Sehachse liegt und der Wert der Y-Achse nivelliert ist, wird der Benutzer angewiesen, die Vorrichtung translatorisch nach unten zu bewegen; und
wurde erkannt, dass die Kamera über der Sehachse liegt und der Wert der Y-Achse nicht nivelliert ist, wird der Benutzer angewiesen, die Vorrichtung nach unten zu kippen.

2. Verfahren nach dem vorangehenden Anspruch, wobei die genannte Vorrichtung eine NIR-, nahes Infrarot, Lichtquelle und eine sichtbare Weißlichtquelle umfasst, und das genannte Verfahren ferner die Verwendung der genannten NIR-Lichtquelle für eine nichtmiotische Hintergrundbeleuchtung umfasst, während die Kamera auf die Sehachse der Netzhaut ausgerichtet wird, und die Verwendung der genannten sichtbaren Weißlichtquelle für die Hintergrundbeleuchtung zur Bilderfassung der Netzhaut umfasst, wenn die Kamera auf die Sehachse der Retina ausgerichtet ist.

3. Verfahren nach einem der vorangehenden Ansprüche, ferner umfassend:
Verwendung eines Verfahrens zur Klassifizierung von Bildern, das durch ein Vortraining mit zuvor erfassten Bildern konfiguriert wurde, um zu erkennen, ob die Kamera auf die Sehachse der Netzhaut ausgerichtet ist; und
Auslösen der Bilderfassung der Netzhaut, wenn die Ausrichtung erkannt wird.

4. Verfahren nach einem der vorangehenden Ansprüche, ferner umfassend die Verwendung eines Verfahrens zur Klassifizierung von Bildern, das durch ein Vortraining mit zuvor erfassten Bildern konfiguriert wurde, um zu erkennen, ob die Kamera falsch nach links oder rechts auf die Sehachse der Netzhaut ausgerichtet ist, indem erkannt wird, ob auf der linken bzw. rechten Seite des erfassten Bildes ein dunkler Schatten vorhanden ist;
wurde erkannt, dass die Kamera falsch nach links ausgerichtet ist, wird der Benutzer angewiesen, die Vorrichtung nach rechts zu drehen; und
wurde erkannt, dass die Kamera falsch nach rechts ausgerichtet ist, wird der Benutzer angewiesen, die Vorrichtung nach links zu drehen.

5. Verfahren nach einem der vorangehenden Ansprüche, wobei das Vortraining mit zuvor erfassten Bildern des Verfahrens zur Klassifizierung von Bildern ein maschinelles Lernverfahren ist.

6. Tragbare optische Vorrichtung zur Erfassung nicht-mydriatischer Netzhautbilder eines Patienten unter Verwendung eines Smartphones mit einer Kamera, einem Trägheitssensor und einem elektronischen Datenprozessor, wobei die genannte Vorrichtung das genannte Smartphone, ein Gehäuse zum Verbinden mit dem Smartphone und ein optisches System zum Erfassen von Bildern der Netzhaut durch die genannte Kamera umfasst, wobei das genannte Smartphone einen nichtflüchtigen Datenträger umfasst, der Anweisungen enthält, die von dem genannten elektronischen Datenprozessor gelesen werden können, die, wenn sie ausgeführt werden, den Prozessor dazu veranlassen, die folgenden Schritte zum Ausrichten der Kamera auf die Sehachse der Netzhaut durchzuführen:
Erfassung eines Bildes der Netzhaut;
Verwendung eines Verfahrens zur Klassifizierung von Bildern, das durch ein Vortraining mit zuvor erfassten Bildern konfiguriert wurde, um zu erkennen, ob die Kamera über oder unter der Sehachse der Netzhaut liegt, indem erkannt wird, ob auf dem erfassten Bild oben bzw. unten ein dunkler Schatten vorhanden ist;
wurde erkannt, dass die Kamera über oder unter der Sehachse der Netzhaut liegt, wird der Wert der Y-Achsenkomponente des Trägheitssensors eingegeben;
wurde erkannt, dass die Kamera unter der Sehachse liegt und der Wert der Y-Achse nivelliert ist, wird der Benutzer angewiesen, die Vorrichtung translatorisch nach oben zu bewegen;
wurde erkannt, dass die Kamera unter der Sehachse liegt und der Wert der Y-Achse nicht nivelliert ist, wird der Benutzer angewiesen, die Vorrichtung nach oben zu kippen;
wurde erkannt, dass die Kamera über der Sehachse liegt und der Wert der Y-Achse nivelliert ist, wird der Benutzer angewiesen, die Vorrichtung translatorisch nach unten zu bewegen;
wurde erkannt, dass die Kamera über der Sehachse liegt und der Wert der Y-Achse nicht nivelliert ist, wird der Benutzer angewiesen, die Vorrichtung nach unten zu kippen.

7. Vorrichtung nach dem vorangehenden Anspruch, umfassend eine NIR-, nahes Infrarot, Lichtquelle zur nicht-miotischen Hintergrundbeleuchtung, während die Kamera auf die Sehachse der Netzhaut ausgerichtet wird.

8. Vorrichtung nach einem der Ansprüche 6-7, umfassend eine sichtbare Weißlichtquelle zur Hintergrundbeleuchtung für die Bilderfassung der Netzhaut.

9. Vorrichtung nach einem der Ansprüche 6-8, umfassend eine sichtbare Lichtquellenmatrix, die so angeordnet ist, dass sie visuelle Fixierpunkte für den Patienten durch das genannte optische System bereitstellt.

10. Vorrichtung nach dem vorangehenden Anspruch, umfassend eine Linse mit einer planen Oberfläche und einer konvexen Oberfläche zum Bündeln von Lichtstrahlen, die von der Matrix der sichtbaren Lichtquelle kommen und in das genannte optische System gehen.

11. Vorrichtung nach einem der Ansprüche 7, 8 und einem der Ansprüche 9-10, umfassend einen Strahlenteiler zur Trennung der genannten visuellen Fixierpunkte der NIR- und der sichtbaren Lichtquellen zur Beleuchtung.

12. Vorrichtung nach einem der Ansprüche 6-11, umfassend einen Strahlenteiler zur Trennung der genannten NIR- und der sichtbaren Lichtquelle der Kamera für die Bilderfassung.

13. Vorrichtung nach einem der Ansprüche 6-12, umfassend:
eine asphärische Linse zur Bildfokussierung unter Vermeidung optischer Aberrationen und/oder
eine Okularlinse, wobei die Oberflächen beider Linsen gekrümmt sind, und/oder
eine Irisblende zur Begrenzung des von dem genannten optischen System erfassten, von dem Augenhintergrund kommenden Lichts.

14. Vorrichtung nach einem der Ansprüche 6-13, umfassend eine oder mehrere Polarisatoren.

15. Vorrichtung nach einem der Ansprüche 6-14, wobei das genannte Gehäuse 3D-gedruckt ist, um zu einer bestimmten Form eines Smartphones zu passen.

## Revendications

1. Procédé d'opération d'un dispositif optique portatif de capture d'images rétiniennes non mydriatiques chez un patient à l'aide d'un smartphone ayant une caméra, un capteur inertiel et un traiteur de données électronique, ledit dispositif comprenant un logement pour accoupler au smartphone et un système optique de capture d'images de rétine à travers ladite caméra, ledit procédé comprenant, pour aligner la caméra avec l'axe visuel de la rétine, la réalisation, par le traiteur de données électronique, des étapes suivantes :
capturer une image de la rétine ;
utiliser un procédé de classification d'image configuré par pré-entrainement sur des images préalablement capturées pour détecter si la caméra est plus basse ou plus haute que l'axe visuel de la rétine, en détectant si une ombre foncée est présente en haut ou en bas, respectivement, de l'image capturée ;
si la caméra a été détectée comme étant plus basse ou plus haute que l'axe visuel de la rétine, insérer la valeur du composant de l'axe Y du capteur inertiel ;
si la caméra a été détectée comme étant plus basse que l'axe visuel et la valeur de l'axe Y est nivelée, informer l'utilisateur qu'il doit mouvoir le dispositif en translation vers le haut ;
si la caméra a été détectée comme étant plus basse que l'axe visuel et la valeur de l'axe Y n'est pas nivelée, informer l'utilisateur qu'il doit incliner le dispositif vers le haut ;
si la caméra a été détectée comme étant plus haute que l'axe visuel et la valeur de l'axe Y est nivelée, informer l'utilisateur qu'il doit mouvoir le dispositif en translation vers le bas ; et
si la caméra a été détectée comme étant plus haute que l'axe visuel et la valeur de l'axe Y n'est pas nivelée, informer l'utilisateur qu'il doit incliner le dispositif vers le bas.

2. Procédé selon la revendication précédente, dans lequel ledit dispositif comprend une source lumineuse NIR, proche infrarouge, et une source lumineuse blanche visible, et ledit procédé comprend également utiliser ladite source lumineuse NIR pour une illumination du fond d'oeil non myotique tout en alignant la caméra avec l'axe visuel de la rétine et comprend utiliser ladite source lumineuse blanche visible pour une illumination du fond d'oeil pour l'acquisition d'une image de la rétine lorsque la caméra est alignée avec l'axe visuel de la rétine.

3. Procédé selon l'une quelconque des revendications précédentes comprenant également :
utiliser un procédé de classification d'image configuré par pré-entrainement sur des images préalablement capturées pour détecter si la caméra est alignée avec l'axe visuel de la rétine ; et
déclencher la capture d'image de rétine lorsque l'alignement est détecté.

4. Procédé selon l'une quelconque des revendications précédentes comprenant également utiliser un procédé de classification d'image configuré par pré-entrainement sur des images préalablement capturées pour détecter si la caméra est désalignée vers la gauche ou vers la droite de l'axe visuel de la rétine, en détectant si une ombre foncée est présente sur la gauche ou sur la droite, respectivement, de l'image capturée ;
si la caméra a été détectée comme étant désalignée vers la gauche, informer l'utilisateur qu'il doit pivoter le dispositif vers la droite ; et
si la caméra a été détectée comme étant désalignée vers la droite, informer l'utilisateur qu'il doit pivoter le dispositif vers la gauche.

5. Procédé selon l'une quelconque des revendications précédentes, dans lequel le pré-entrainement sur des images préalablement capturées du procédé de classification d'image est un procédé d'apprentissage machine.

6. Dispositif optique portatif pour capturer des images rétiniennes non mydriatiques d'un patient à l'aide d'un smartphone ayant une caméra, un capteur inertiel et un traiteur de données électronique, ledit dispositif comprenant ledit smartphone, un logement pour accoupler au smartphone et un système optique pour capturer des images de rétine à travers ladite caméra, ledit smartphone comprenant un support de données non volatile comprenant des instructions lisibles par ledit traiteur de données électronique qui, lorsqu'elles sont exécutées, amènent le traiteur à exécuter les étapes suivantes pour aligner la caméra avec l'axe visuel de la rétine :
capturer une image de la rétine ;
utiliser un procédé de classification d'image configuré par pré-entrainement sur des images préalablement capturées pour détecter si la caméra est plus basse ou plus haute que l'axe visuel de la rétine, en détectant si une ombre foncée est présente sur le haut ou sur le bas, respectivement, de l'image capturée ;
si la caméra a été détectée comme étant plus basse ou plus haute que l'axe visuel de la rétine, insérer la valeur du composant de l'axe Y du capteur inertiel ;
si la caméra a été détectée comme étant plus basse que l'axe visuel et la valeur de l'axe Y est nivelée, informer l'utilisateur qu'il doit mouvoir le dispositif en translation vers le haut;
si la caméra a été détectée comme étant plus basse que l'axe visuel et la valeur de l'axe Y n'est pas nivelée, informer l'utilisateur qu'il doit incliner le dispositif vers le haut ;
si la caméra a été détectée comme étant plus haute que l'axe visuel et la valeur de l'axe Y est nivelée, informer l'utilisateur qu'il doit mouvoir le dispositif en translation vers le bas ;
si la caméra a été détectée comme étant plus haute que l'axe visuel et la valeur de l'axe Y n'est pas nivelée, informer l'utilisateur qu'il doit incliner le dispositif vers le bas.

7. Dispositif selon la revendication précédente comprenant une source lumineuse NIR, proche infrarouge, pour l'illumination du fond d'oeil non myotique tout en alignant la caméra avec l'axe visuel de la rétine.

8. Dispositif selon l'une quelconque des revendications 6-7 comprenant une source lumineuse blanche visible pour l'illumination du fond d'oeil pour l'acquisition d'une image de la rétine.

9. Dispositif selon l'une quelconque des revendications 6-8 comprenant une matrice de source lumineuse visible arrangée pour fournir des points de fixation visuels pour le patient à travers ledit système optique.

10. Dispositif selon la revendication précédente comprenant un objectif ayant une surface planaire et une surface convexe pour condenser les rayons de lumière venant de la matrice de source lumineuse visible dans ledit système optique.

11. Dispositif selon les revendications 7, 8 et l'une quelconque des revendications 9-10 comprenant un séparateur de faisceau pour séparer lesdits points de fixation visuels du NIR et les sources lumineuses d'illumination visibles.

12. Dispositif selon l'une quelconque des revendications 6-11 comprenant un séparateur de faisceau pour séparer ledit NIR et les sources lumineuses d'illumination visibles de la caméra de capture d'images.

13. Procédé selon l'une quelconque des revendications 6-12 comprenant :
un objectif asphérique destiné à la mise au point d'une image tout en évitant les aberrations optiques, et/ou
un objectif oculaire ayant deux surfaces du objectif courbées, et/ou
un iris pour limiter la lumière capturée par ledit système optique à la lumière venue du fond d'œil.

14. Dispositif selon l'une quelconque des revendications 6-13 comprenant un ou plusieurs polariseurs de lumière.

15. Dispositif selon l'une quelconque des revendications 6-14 dans lequel ledit logement est imprimé en 3D pour recevoir un format de smartphone spécifique.
